# EUROPEAN PATENT SPECIFICATION

(11) **EP 1 018 866 B1**
(45) Date of publication and mention of the grant of the patent: **04.12.2002**
(21) Application number: 96918938.0
(22) Date of filing: 14.06.1996
(51) Int. Cl.: A01N 1/00, A01N 1/02, A01N 3/00, C12N 1/04, C12N 5/00, A61K 35/12, A61K 35/14

(54) **COMPOSITIONS AND METHODS FOR THE PRESERVATION OF LIVING TISSUES**
ZUSAMMENSETZUNGEN UND VERFAHREN ZUM KONSERVIEREN VON LEBENDEN GEWEBEN
COMPOSITIONS ET PROCEDES DE CONSERVATION DE TISSUS VIVANTS

(43) Date of publication of application: 19.07.2000
(73) Proprietor: Biostore New Zealand Limited, Parnell, Auckland (NZ)
(72) Inventor: WIGGINS, Philippa, M., North Shore City, Auckland (NZ); FERGUSON, Alexander, B., Auckland 5 (NZ)
(74) Representative: Patentanwälte Feldmann & Partner AG
(86) International application number: NZ9600057
(87) International publication number: WO97047192

(56) References cited:
- EP-A- 0 099 315
- EP-A- 0 259 739
- EP-A- 0 306 132
- EP-A- 0 312 114
- EP-A- 0 556 096
- WO-A-91/18504
- WO-A-92/03046
- WO-A-92/18136
- WO-A-93/04578
- WO-A-93/14191
- DD-A- 228 439
- DE-C- 3 625 170
- US-A- 4 380 582
- US-A- 5 242 792
- DATABASE WPI Week 9209, Derwent Publications Ltd., London, GB; Class D22, AN 1992-065217, XP002944377 & BR 9 003 310 A (EMBRAPA EMPRESA BRA) 28 January 1992
- ANIMAL REPRODUCTION SCIENCE, (1994), 36(1-2), F.C. MOLINIA et al., "Effect of Monosaccharides and Disaccharides in Tris-Based Diluents on Motility, Acrosome integrity and Fertility of Pellet Frozen Ram Spermatozoa", pages 113-122, XP001008237.
- BIOCHEM. AND BIOPHYS. RESEARCH COMMUN., (1989), 164(3), S. YANO and D.F. TIERNEY, "Butyrate Increases Catalase Activity and Protects Rat Pulmonary Artery Smooth Muscle Cells Against Hyperoxia", pages 1143-1148, XP001008338.
- JOURNAL OF CELLULAR PHYSIOLOGY, (1988), 135(3), J.L. STAECKER et al., "Sodium Butyrate Preserves Aspects of the Differentiated Phenotype of Normal Adult Rat Hepatocytes in Culture", pages 367-376, XP000617246.
- J. FOOD BIOCHEM., (1987), 11(2), J.W. PARK et al., "Effects of Cryoprotectants in Minimising Physiochemical Changes of Bovine Natural Actomyosin During Frozen Storage", pages 143-161, XP001007469.
- CRYOBIOLOGY, (1994), 31(4), P. BOUTRON and J.F. PEYRIDIEU, "Reduction in Toxity for Red Blood Cells in Buffered Solutions Containing High Concentrations of 2,3-Butanediol by Trehalose, Sucrose, Sorbitol or Mannitol", pages 367-373, XP001008337.
- CRYOBIOLOGY, (1992), 29, A.M. KAROW et al., "Effects of Temperature, Potassium Concentration and Sugar on Human Spermatazoa Motility: A Cell Preservation Model from Reproductive Medicine", pages 250-254, XP001007475.
- BIOTECHNOLOGY AND GENETIC ENGINEERING REVIEWS, (1993), 11, Y.M. NEWMAN et al., "The Role of Trehalose and Other Carbohydrates in Biopreservation", pages 263-294, XP000570019.
- VOX SANGUINIS, (1981), 41(5-6), C.F. HOEGMAN et al., "Red Cell Preservation in Protein-Poor Media", pages 274-281, XP001008334.
- FOLIA HAEMATOLOGICA, INTERNATIONALES MAGAZIN FUER KLINISCHE UND MORPHOLOGISCHE HAMATOLOGIE, (1987), 114(4), D. STIBENZ et al., "Preservation of Resuspended Red Cell Concentrates. Rate of Vesiculation and of Spontaneous Hemolysis", pages 469-470, XP002944388.
- CRYOBIOLOGY, (1988), 25(2), W.T. SHIER, "Studies on the Mechanism of Mammalian Cell Killing by a Freeze-Thaw Cycle: Conditions that Prevent Cell Killing Using Nucleated Freezing", pages 110-120, XP001007474.
- BIOCHEMICAL SOCIETY TRANSACTIONS, (1988), 16(3), C.A. BREARLEY et al., "A Comparative Study of the Cryopreservation of Human Erythrocytes, Ghosts and Liposomes", page 354, XP001008332.
- NUCLEIC ACID RESEARCH, (1989), 17(10), S. GOLDSTEIN et al., "Enhanced Transfection Efficiency and Improved Cell Survival After Electroporation of G2/M - Synchronized Cells and Treatment with Sodium Butyrate", pages 3959-3971, XP002054817.
- DATABASE WPI Week 9502, Derwent Publications Ltd., London, GB; Class E16, AN 1995-009512, XP002944378 & JP 6 293 602 A (SHINKO SANGYO KK) 21 October 1994
- COMPTES RENDUS DE L'ACADEMIE DES SCIENCES SERIE III, SCIENCES DE LA VIE, (1987), 304(20), J. FABRE and J. DEREUDDRE, "Effects of Different Substances (Sucrose, Glucose, Sorbitol and Mannitol) on the Resistance to Deep Freezing in Liquid Nitrogen of Meristems from in Vitro Cultured Carnations", pages 507-510, XP001008206.
- GASTROENTEROLOGY, (1993), 105(5), C.A. BRASS et al., "Evaluation of University of Wisconsin Cold-Storage Solution in Warm Hypoxic Perfusion of Rat Liver: the Addition of Fractose Reduces Injury", pages 1455-1463, XP001011520.
- THE ANNALS OF THORACIC SURGERY, (November 1994), 58(5), S.F. FREMES et al., "Cardiac Storage with UW Solution and Glucose", pages 1368-1372, XP001008339.
- TRANSPLANTATION PROCEEDINGS, (August 1995), 27(4), P. ESCHWEGE et al., "Successful -4°C Liver Preservation in Rats with University of Wisconsin Solution and 2-3-Butanediol", pages 2514-2515, XP001007470.
- TRANSPLANTATION INTERNATIONAL, (1995), 8(3), T. MINOR et al., "Effects of Taurine on Liver Preservation in UW Solution with Consecutive Ischemic Rewarming in the Isolated Perfused Rat Liver", pages 174-179, XP002944389.
- INDIAN JOURNAL OF ANIMAL HEALTH, (1983), 22(2), K. AHMAD and R.A. CHAUDHRY, "Deep Freezing of Buffalo Bull Semen of Nili-ravi Breed", pages 111-114, XP002944390.
- IN VITRO CELL DEVEL. BIOL., (May 1995), 31(5), W.T. SHIER and S.G. OLSEN, "Isotonic Sucrose Improves Cryopreservation of Cultured Mammalian Cells", pages 336-337, XP002944398.
- BIOMED. BIOCHIM. ACTA, (1987), 46, D. STRAUSS et al., "SAG-Sucrose Medium for Red Blood Cell Preservation", pages 295-299, XP001008234.
- DATABASE WPI Week 199505, Derwent Publications Ltd., London, GB; AN 1995-036643, XP002942623 & WO 94 29691 A1 (COULTER CORP) 22 December 1994

## Description

### Technical Field of the Invention

This invention relates to the field of preservation of biological materials and, more particularly, to compositions and methods for the preservation of organs, tissues and cells from mammals, marine organisms and plants.

### Background of the Invention

Methods for the preservation of biological materials are employed in many clinical and veterinary applications wherein living material, including organs, tissues and cells, is harvested and stored *in vitro* for some period of time before use. Examples of such applications include organ storage and transplants, autologous and allogeneic bone marrow transplants, whole blood transplants, platelet transplants, embryo transfer, artificial insemination, *in vitro* fertilization, skin grafting and storage of tissue biopsies for diagnostic purposes. Preservation techniques are also important in the storage of cell lines for experimental use in hospital, industrial, university and other research laboratories.

Methods currently employed for the preservation of cellular biological materials include immersion in saline-based media; storage at temperatures slightly above freezing; storage at temperatures of about -80 °C, and storage in liquid nitrogen at temperatures of about -196 °C. The goal of these techniques is to store living biological materials for an extended period of time with minimal loss of normal biological structure and function.

Storage of organs, such as heart and kidneys, at temperatures below 0 °C frequently results in the loss of many cells with a corresponding reduction in viability of the organ. Such complex biological materials are therefore typically stored in aqueous, saline-based media at temperatures above freezing, typically around 4 °C Saline-based media typically consist of isotonic saline (sodium chloride 0.154 M) which has been modified by the addition of low concentrations of various inorganic ions, such as sodium, potassium, calcium, magnesium, chloride, phosphate and bicarbonate, to mimic the extracellular environment. Small amounts of compounds such as glucose, lactose, amino acids and vitamins are often added as metabolites. All saline-based media used for preservation of biological materials have high electrical conductivity. Examples of media currently employed for the preservation of biological materials include phosphate-buffered saline (PBS), M-2 (a Hepes buffered murine culture medium), Ringer's solution and Krebs bicarbonate-buffered medium.

The viability of biological materials stored in saline-based media gradually decreases over time. Loss of viability is believed to be due to the build-up of toxic wastes, and loss of metabolites and other supporting compounds caused by continued metabolic activity. Using conventional saline-based media, living tissues can only be successfully preserved for relatively short periods of time. Examination of the microstructure of organs stored towards the upper limit of time shows degeneration, such as of mitochondria in heart muscle, and the performance of the organ once replaced is measurably compromised. For example, a human heart can only be stored in cold ionic solutions for about 5 hours following removal from a donor, thereby severely limiting the distance over which the heart can be transported.

When employing freezing techniques to preserve biological materials, cryoprotectants, such as glycerol, dimethylsulfoxide (DMSO), glycols or propanediol, are often introduced to the material prior to freezing in order to limit the amount of damage caused to cells by the formation of ice crystals during freezing. The choice and concentration of cryoprotectant, time-course for the addition of cryoprotectant and temperature at which the cryoprotectant is introduced all play an important role in the success of the preservation procedure. Furthermore, in order to reduce the loss of cells, it is critical that such variables as the rate and time-course of freezing, rate and time-course of thawing and further warming to room or body temperature, and replacement of cryoprotectant solution in the tissue mass with a physiological saline solution be carefully controlled. The large number of handling steps required in freezing techniques increases the loss of cells. The freezing techniques currently employed in the preservation of biological materials are both technically demanding and time consuming. Other disadvantages of preserving biological materials by freezing include: reduction of cell viability; potential toxic effects of the cryoprotectant to the patient upon re-infusion; and the high costs of processing and storage.

As an example, cryopreservation, generally including the addition of DMSO as a cryoprotectant, is presently used to store bone marrow harvested for use in transplantation procedures following, for example, high dose chemotherapy or radiotherapy. In autologous transplants the bone marrow must be preserved for prolonged periods, ranging from weeks to months. However, this technique results in significant reduction of stem cell recovery, to levels as low as 50% or less. An additional disadvantage of this technique is that significant damage to various mature cells can occur, thereby requiring further processing to remove these cells prior to freezing. Finally, the use of DMSO results in moderate to severe toxicity to the patient on re-infusion of the preserved bone marrow.

There thus remains a need in the art for improved methods for the preservation of living biological materials.

### Summary

The present invention provides compositions and methods for preserving biological materials that enable materials including organs, tissues and cells to be stored for extended periods of time with minimal loss of biological activity.

In a first aspect, the invention provides solutions which are substantially free of univalent oxyanions and of iodide, and which are substantially isotonic with the biological material to be preserved; Such solutions include a first neutral solute with no net charge, having a molecular weight of at least about 335 and a solubility in water of at least about 0.3 M; and a second neutral solute having a molecular weight of less than about 200, the second solute additionally having both hydrophilic and hydrophobic moieties.

Preservation solutions of the present invention may also include one or more ions. A calcium salt, preferably CaSO₄ or CaCl₂, is used at concentrations of below about 2 mM in many types of preservation solutions. Additional ions may be selected according to their characteristic position in the Hofmeister series of anions or cations. Specifically, ions from the protein-stabilizing ends of the Hofmeister series may be included in the preservation solutions of the present invention. Appropriate ions and selection criteria are described in detail below.

In one embodiment, the first neutral solute is either a disaccharide or a trisaccharide, preferably selected from the group consisting of raffinose, trehalose, sucrose, lactose and analogs thereof. The analogs may be either naturally occurring or synthetic. The second neutral solute is preferably selected from the group consisting of trimethyl amino oxide (TMAO), betaine, taurine, sarcosine, glucose, mannose, fructose, ribose, galactose, sorbitol, mannitol, inositol and analogs thereof. The inventive solutions may also comprise sodium sulfate and calcium, the calcium preferably being present as calcium sulfate at a concentration of about 1.5 mM to about 2.0 mM, most preferably about 1.75 mM.

While the preferred solution for the preservation of a biological material will depend upon the specific biological material to be preserved, it has been found that solutions comprising either raffinose and TMAO, or trehalose and TMAO are particularly efficacious in the preservation of many biological materials. In one embodiment, the inventive solutions comprise raffinose and TMAO in a ratio of about 1.4:1 to about 1.8:1, preferably 1.6:1. Preferably, the solutions of the present invention comprise between about 60% and about 80% raffinose and TMAO, between about 40% and about 20% sodium sulfate, and between about 1.5 mM and about 2.0mM calcium sulfate, the raffinose and TMAO being present in a ratio of about 1.6:1. Most preferably, the solution comprises about 70% raffinose and TMAO, about 30% sodium sulfate and about 1.75 mM calcium sulfate, the raffinose and TMAO being present in a ratio of about 1.6:1. In a second embodiment, the solutions comprise trehalose and TMAO in a ratio of between about 1.1:1 and about 1.4:1, most preferably in a ratio of 1.3:1, with the addition of calcium sulfate.

The osmolality of the preservation solutions of the present invention varies with the biological material to be preserved, with an osmolality of about 0.28 to about 0.32 OsM being preferred for mammalian biological materials, an osmolality of about 70 to about 80 mOsM being preferred for plant biological materials, and an osmolality of about 0.9 to about 1.0 OsM being preferred for marine biological materials.

In a related aspect, the present invention provides dehydrated and concentrated forms of the inventive preservative solutions. Such forms may be either solid, such as a powder or tablet, or liquid.

In another aspect, the present invention provides methods for the preservation of biological materials, including mammalian, plant and marine materials, comprising contacting the biological material with one or more of the inventive solutions.

In yet another aspect, a method for the treatment of leukemia is provided, the method comprising removing bone marrow from a patient, contacting the bone marrow with a preservation composition or solution of the present invention for a period of at least about 3 days in order to purge the bone marrow of leukemic cells, and returning the purged bone marrow to the patient.

As detailed below, it has been found that the solutions and methods of the present invention can be employed to maintain the viability of biological materials, including cells, tissues and organs, for longer periods of time than are generally possible with conventional preservation methods, thereby providing improved storage and transport times for biological materials for use in applications such as organ transplants and bone marrow transplants.

The preservation methods of the present invention are less complex than many of the methods typically employed for the preservation of biological materials, thereby reducing costs and increasing the ease of use and availability of preservation procedures. Furthermore, the inventive compositions are of low toxicity, resulting in fewer negative side effects when biological materials, such as transplant organs, are returned to a patient.

The above-mentioned and additional features of the present invention and the manner of obtaining them will become apparent, and the invention will be best understood by reference to the following more detailed description, read in conjunction with the accompanying drawings.

### Brief Description of the Drawings

Figs. 1A, B and C illustrate the survival of mouse embryos following 1, 2 and 3 days of storage, respectively, at 4 °C in an aqueous solution of sucrose and various Class II solutes, together with 1.75 mM CaSO₄.
Figs. 2A, B and C illustrate the survival of mouse embryos following 1, 2 and 3 days of storage, respectively, at 4 °C in an aqueous solution of lactose and various Class II solutes, together with 1.75 mM CaSO₄.
Figs. 3A, B and C illustrate the survival of mouse embryos following 1, 2 and 3 days of storage, respectively, at 4 °C in an aqueous solution of trehalose and various Class II solutes, together with 1.75 mM CaSO₄.
Figs. 4A, B and C illustrate the survival of mouse embryos following 1, 2 and 3 days of storage, respectively, at 4 °C in an aqueous solution of raffinose and various Class II solutes, together with 1.75 mM CaSO₄.
Figs. 5A, B and C illustrate the survival of mouse embryos after storage for 1, 2 and 3 days, respectively, at 4 °C in aqueous solutions with varying molar ratios of raffinose to TMAO, with 1.75 mM CaSO₄.
Fig. 6 shows the Ca²⁺ dependence of mouse embryo survival following storage in raffinose/TMAO at 4 °C for 2 and 3 days.
Figs. 7(i)A, B and C show the survival of mouse embryos following storage for 1, 2 and 3 days, respectively, at 4 °C in mixtures of raffinose/TMAO and Na₂SO₄, with 1.75 mM CaSO₄. Fig. 7(ii) shows the mean and SEM of survival of mouse embryos following 1, 2, 3 and 4 days of storage in Solution 70/30 at various osmolalities.
Figs. 8A, B and C show the percentage of mouse embryos reaching the late blastocyst stage following storage for 1, 2 and 3 days, respectively, at 4 °C in Solution 70/30 after pretreatment with 5, 10 or 15 mM sodium butyrate in PBS at room temperature for 10, 20 or 30 minutes. Figs. 8D, E and F show the percentage of mouse embryos alive following storage for 1, 2 and 3 days, respectively, at 4 °C in Solution 70/30 after pretreatment with 5, 10 or 15 mM sodium butyrate in PBS at room temperature for 10, 20 or 30 minutes.
Fig. 9 shows the survival of mouse embryos following storage at 4 °C in PBS, raffinose/TMAO (ratio 1.6:1), or Solution 70/30, with and without pretreatment with 70 mM butyrate in PBS.
Figs. 10A, B, C and D show the survival of mouse embryos after storage in Solution 70/30 for 1, 2, 3 and 4 days following pretreatment with 25 mM sodium butyrate in PBS for 5, 10, or 15 minutes.
Fig. 11 shows the survival of platelets following storage at 4 °C in either plasma or Ca²⁺-free Solution 70/30.
Figs. 12A, B and C show the number of CD45- and CD34-positive cells and colony forming units, respectively, in bone marrow from patient 1 following storage in either Hanks buffered saline solution, raffinose/TMAO, trehalose/betaine or Solution 70/30.
Figs. 13A, B and C show the percentage of colony forming units, CD34- and CD45-positive cells, respectively, in bone marrow from patient 2 following storage in either raffinose/TMAO with 1.75 mM CaSO₄ or in M-2 at 4 °C.
Fig. 14A shows the percentage recovery of murine bone marrow cells stored in PBS or Solution 70/30 at either 4°C or -80°C. Fig. 14B shows the number of cell colonies found in spleens of lethally irradiated mice 8 days after injection of thawed murine bone marrow cells which had been stored in Solution 70/30 for 8 days at -80 °C.
Fig. 15 shows a trace from a pressure transducer for a rat heart following storage for 4 hours at 4 °C in Solution 70/30.
Fig. 16 shows the proliferation of Jurkat cells (acute T-cell leukemia) assessed by uptake of tritiated thymidine, following storage at 4 °C in saline or in preservation solutions of the present invention for up to 48 hours.
Fig. 17 shows proliferation of K562 chronic myelogenous leukemia cells assessed by uptake of tritiated thymidine following storage at 4°C in saline or in preservation solutions of the present invention for up to 48 hours.
Fig. 18 shows proliferation to confluence of murine osteoblasts following storage at 4 °C in PBS and various preservation solutions of the present invention.
Fig. 19 shows the percentage recovery of a murine keratocyte cell line T7T after storage in PBS or Solution 70/30.

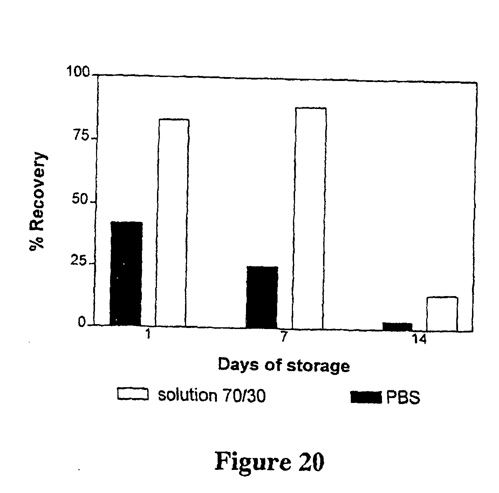

Fig. 20 shows the percentage recovery of murine 3T3 fibroblasts after storage in PBS or Solution 70/30.

### Detailed Description

The solutions and methods of the present invention may be used in the preservation of biological materials including mammalian, plant and marine cells, cell lines, tissues and organs. When a biological material is preserved., its viability is maintained *in vitro* for an extended period of time, such that the material resumes its normal biological activity on being removed from storage. During storage the biological material is thus maintained in a reversible state of dormancy, with metabolic activity being substantially lower than normal. For example, hearts are observed to stop beating during storage. Examples of mammalian biological materials which may be preserved using the present invention include organs, such as heart; cells and tissues such as haematopoietic stem cells, bone marrow, embryos, platelets, osteoblasts, spermatozoa; and various animal cell lines established in tissue culture. In addition to the preservation of human biological materials, the inventive solutions and methods may also be employed in veterinary applications, and for preservation of plant tissues.

The preservative solutions of the present invention may be in either a ready-to-use form or may be provided in a concentrated form, such as a solid, including for example, powder or tablets, which is reconstituted in water prior to use. The inventive solutions may also be provided in a concentrated liquid form for dilution by the user. As with conventional preservative solutions, the inventive solutions are sterile.

The solutions of the present invention are substantially isotonic with the biological material to be preserved. Cells in an isotonic solution neither shrink nor swell. While the preservative solutions of the present invention may have an osmolality substantially equal to that of the biological material to be preserved, this is not a requirement of the inventive solutions, since some solutions may include one or more components which raise the osmolality of the solution but are able to cross semi-permeable membranes freely, thus raising the osmotic pressure equally on both sides of the cell membrane.

As detailed below, it has been determined that an osmolality of between about 0.28 OsM and about 0.32 OsM is preferable for solutions for the preservation of mammalian biological materials. Osmolalities of between about 0.9 to about 1.0 OsM and between about 70 to about 80 mOsM are preferred for the preservation of marine and plant biological materials, respectively.

It has been observed that contamination with univalent oxyanions, such as H₂PO₄⁻, HCO₃⁻, NO₃⁻ and HSO₄⁻, increases the level of metabolic activity during storage. For example, contamination with HSO₄⁻ was observed to allow a rat heart to beat slowly and feebly, whereas in the absence of univalent oxyanions, no beating occurs. For most applications, preservative solutions of the present invention exclude univalent oxyanions.

The inventive solutions comprise a first neutral solute having a molecular weight of at least about 335 and a solubility in water of at least about 0.3 M (hereinafter referred to as Class I solutes), and a second neutral solute having a molecular weight of less than about 200 (hereinafter referred to as Class II solutes), the second neutral solute additionally having both hydrophilic and hydrophobic moieties. Class I solutes are generally too large to penetrate cell membranes and act primarily to raise the osmolality of the inventive solutions. Preferably, Class I solutes are disaccharides or trisaccharides. Examples of such solutes include raffinose, trehalose, sucrose, lactose and synthetic or naturally occurring analogs thereof, with raffinose and trehalose being preferred Class I solutes. Class II solutes generally do not passively cross cell membranes, but may be actively taken up by cells in response to an osmotic insult. They are used by many cells as intracellular osmolytes. Examples of such solutes include TMAO, betaine, taurine, sarcosine, glucose, mannose, fructose, ribose, galactose, sorbitol, mannitol and inositol and synthetic or naturally occurring analogs thereof. TMAO is the preferred Class II solute for many biological materials. Betaine is another preferred Class II solute. Solutions comprising either raffinose and TMAO, preferably in a molar ratio of between 1.1:1 to about 2.0:1, more preferably in a molar ratio of between about 1.4:1 to about 1.8:1, most preferably about 1.6:1, or trehalose and TMAO, preferably in a molar ratio of between about 1.1: 1 and about 1.4:1, most preferably about 1.3:1, have been found to be particularly useful for the preservation of biological materials.

The inventive solutions may additionally contain one or more ions but are substantially free of univalent oxyanions and iodide. A calcium salt, such as CaSO₄, is used at concentrations below about 2 mM in preservation solutions for many applications. Other ionic species may be selected according to their characteristic position in the Hofmeister series of anions and/or cations. The Hofmeister series of anions and cations are ranked in order of decreasing stabilization of proteins and membranes (Hofmeister, F., On the understanding of the effect of salts. Second report. On regularities in the precipitating effects of salts and their relationship to their physiological behavior. Naunyn-Schmiedebergs Archiv fuer Experimentalle Pathologic und Pharmakologic. (Leipzig) 14:247-260, 1988; Collins, K.D. and Washabaugh, M W.. The Hofmeister effect and the behavior of water at interfaces. Quarterly. Rev. Biophys. 18:323-422, 1985). The rank order of anions is: citrate > acetate> dihydrogen phosphate> sulfate > hydroxyl > fluoride > chloride > bromide > iodide > hydrogen phosphate > bicarbonate > bisulfate > nitrate. The rank order of cations is: tetramethyl ammonium > ammonium >cesium > rubidium > potassium > sodium > lithium > calcium > magnesium.

Ions, excluding univalent oxyanions and iodide, may be included in preservation solutions of the present invention at concentrations of less than about 2 mM without regard to their rank order. At ionic concentrations of greater than about 2 mM, the following selection criteria apply:
(i) each anion to the left of chloride is combined in a neutral salt with sodium or a cation to the left of sodium;
(ii) anions to the right of chloride are excluded; and
(iii) cations to the right of sodium can be combined only with chloride.
Especially preferred ions for use in preservation solutions includes potassium, tetramethyl ammonium, ammonium, cesium and citrate. Magnesium is also a preferred cation at concentrations of less than about 2 mM.

As detailed below, it has been determined that, with the exception of platelets, effective storage times for biological materials increase with the addition of calcium to the preservative compositions. This may be due to the ability of calcium to stabilize phospholipid bilayers found in cell membranes and to stabilize intercellular adhesion Preferably the calcium is present as calcium sulfate and is present at a concentration of between 1.5 mM and about 2.0 mM, most preferably 1.75 mM. The addition of sodium sulfate also increases effective storage times for many biological materials. A composition comprising between about 60% and about 80%, preferably about 70%, raffinose and TMAO, between about 40% and about 20%, preferably about 30% sodium sulfate, and about 1.75 mM calcium sulfate, with the raffinose and TMAO being present in a ratio of about 1.6:1 has been found to be particularly effective in preserving many biological materials.

Other components which may be included in the inventive compositions include antibiotics for the control of micro-organisms, and proteins, such as bovine serum albumin, for inhibiting the attachment of the biological material, such as embryos, to surfaces. For certain applications, such as storage of hearts, the preservative solution may be saturated with oxygen before use.

While not wishing to be bound by theory, the inventors believe that the preservative solutions of the present invention isolate cells from external stimulatory signals carried through the cell membrane by preventing the opening of ion channels, thereby maintaining the cells in a state of dormancy.

Biological materials to be preserved are harvested using standard techniques and contacted, preferably immersed, in an aqueous preservative solution of the present invention. The biological material may be rinsed with the preservative solution prior to immersion, if required. While the biological materials may be stored at temperatures below freezing, materials are conveniently stored at temperatures of about 4 °C. After storage, the preservative solution may be removed from the material and replaced with a standard saline-based medium or the stored material may be used directly in its preservative solution. When the biological material is stored at temperatures below freezing, an effective concentration of a cryoprotectant may be added to the preservative solution, as employed in techniques well known to those of skill in the art. The inventive solutions may thus be used for either long term or short term storage of biological materials.

As detailed below in Example 2, storage times for some biological materials, such as embryos, may be increased by pretreatment with either a Class II solute or sodium butyrate.

The following examples are offered by way of illustration and not by way of limitation. In the following examples, the use of sucrose and lactose as Class I solutes and the use of class II solutes apart from TMAO and betaine is to be considered comparative.

### Example 1

The efficacy of the solutions of the present invention in the preservation of mouse embryos was tested as described below. As embryos consist of rapidly dividing cells, they are difficult to arrest, and therefore, provide a sensitive test of storage solutions. Embryos also have the advantage that survival in storage can be assessed after 1-5 days by their ability to hatch in subsequent culture.

Viable mouse embryos were stored for periods of 1, 2 or 3 days at 4 °C in either PBS or an aqueous solution of either raffinose, trehalose, sucrose or lactose (Class I solutes), together with a solute selected from the group consisting of trimethyl amine oxide (TMAO), betaine, taurine, sarcosine, glucose, mannose, fructose, ribose, galactose, sorbitol, mannitol. inositol and taurine (Class II solutes), at a ratio of Class I solute to Class II solute of 1.6:1. Each Class I/Class II solution also contained calcium sulfate at a concentration of 1.75 mM. The solutions also contained 0.1-1% bovine serum albumin (BSA) and 25 mg/L of kanamycin sulfate. All reagents were obtained from Sigma Chemical Company (St. Louis, MO). Survival of the embryos was assessed by subsequent culture in Dulbecco's Modified Eagles Medium (DMEM, Life Technologies, Grand Island, New York) and was expressed both as the number of live embryos present after storage and the number of embryos which hatched after 48 hours in culture at 37 °C.

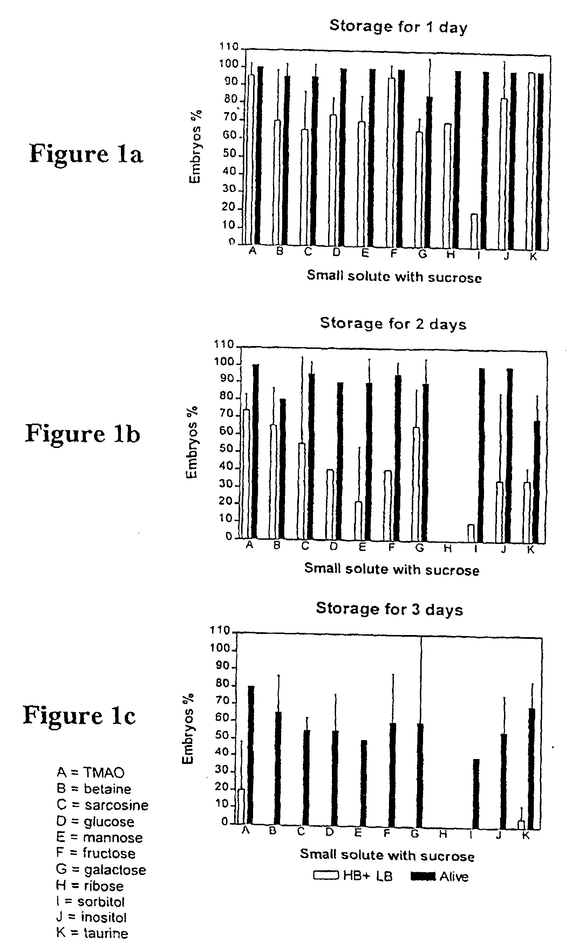

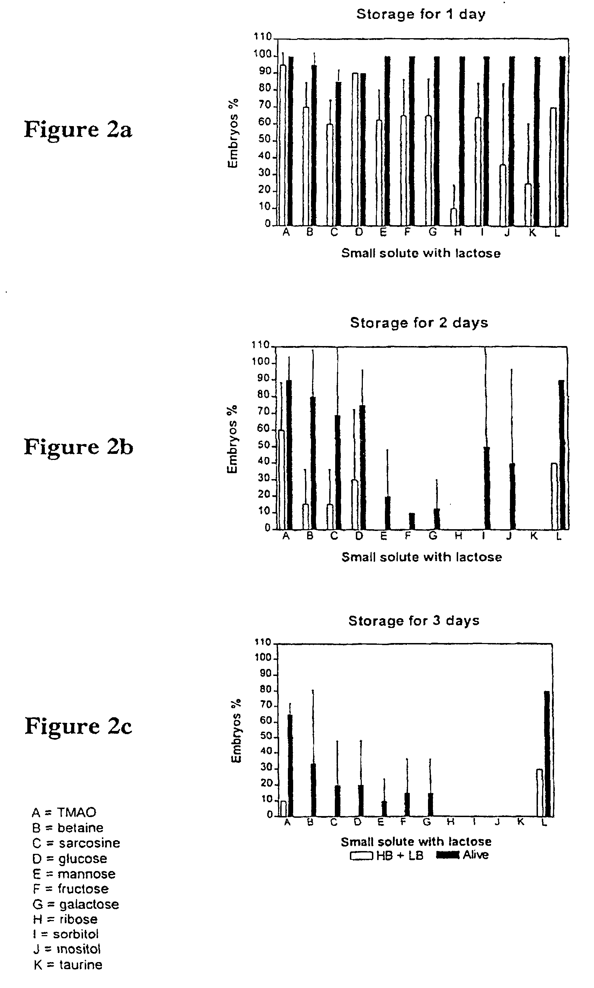

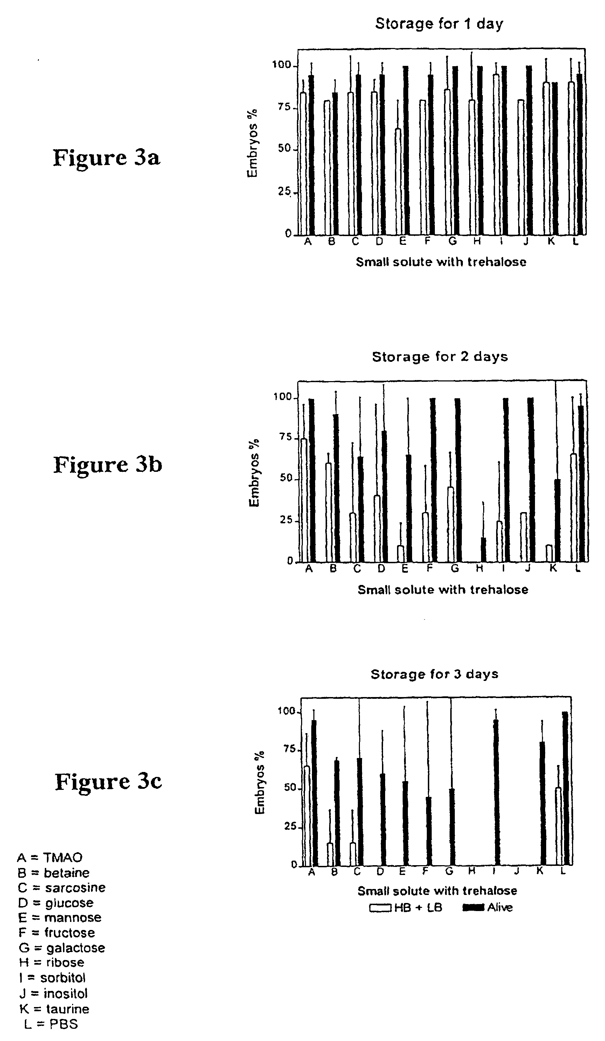

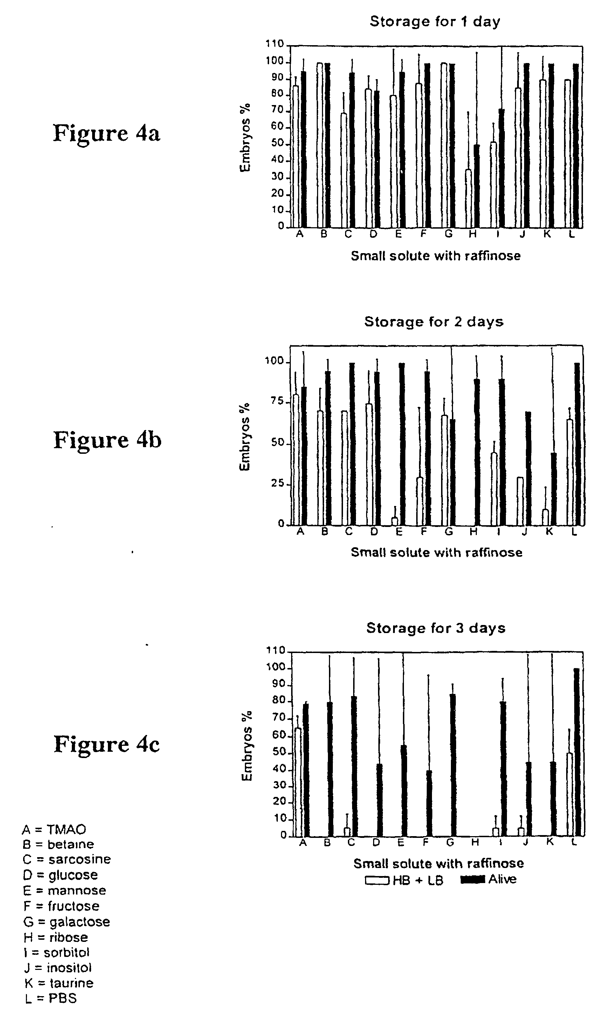

The results of these experiments for solutions of sucrose, lactose, trehalose and raffinose are shown in Figs. 1-4, respectively, wherein HB + LB represents the percentage of embryos hatched or reaching the late blastocyst stage. A significant percentage of embryos hatched following storage for one day in most combinations of solutes, but following three days of storage a high percentage of hatching was only obtained with combinations of raffinose, trehalose or sucrose with TMAO Raffinose was found to be the best Class I solute and TMAO the best Class II solute, with trehalose and betaine being the second best Class I and Class II solutes, respectively. The optimal total osmolality of the Class I/Class II solutions for preservation of mouse embryos was found to be 0.30 OsM.

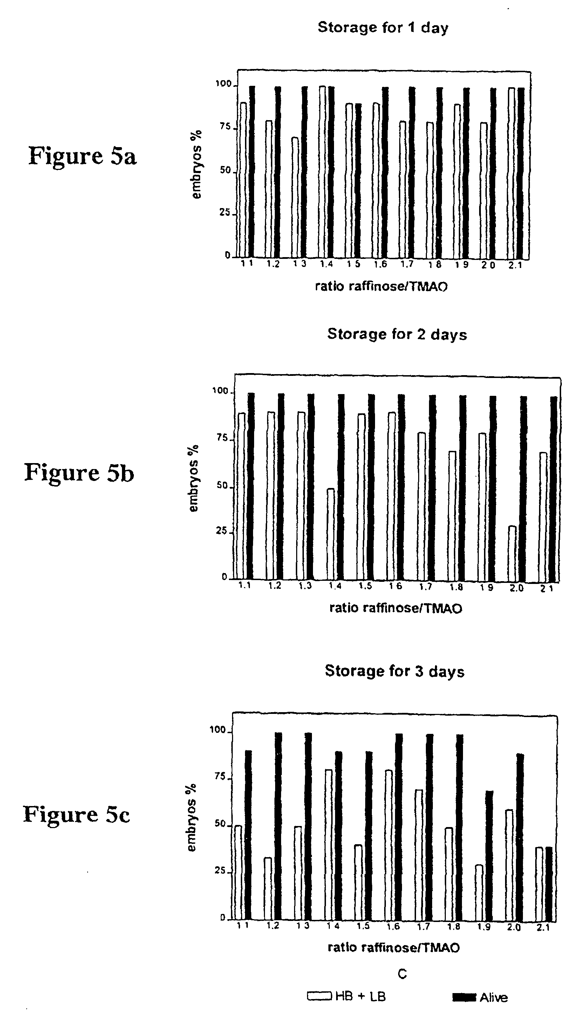

The three best combinations of Class I and Class II solutes were then retested to determine the optimal molar ratios of Class I to Class II solutes. Of these three solutions, a raffinose:TMAO molar ratio of 1.6:1 resulted in the highest percentage of survival of embryos (see Fig. 5). The second highest percentage of survival was obtained with a trehalose:TMAO molar ratio of 1.3:1. The third highest percentage of survival was obtained with a raffinose:betaine molar ratio of 1.4:1.

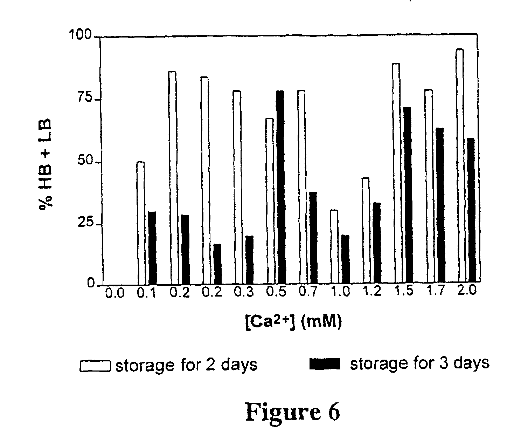

The percentage of embryos hatching following storage in solutions containing a 1.6:1 molar ratio of raffinose to TMAO and varying concentrations of Ca²⁺ is shown in Fig. 6. It was found that Ca²⁺ is required for embryo preservation, with a non-linear concentration dependence. A CaSO₄ concentration of 1.75 mM was subsequently used in all solutions and with most biological materials. One exception was that of isolated platelets which were found to survive best in Ca²⁺-free solutions.

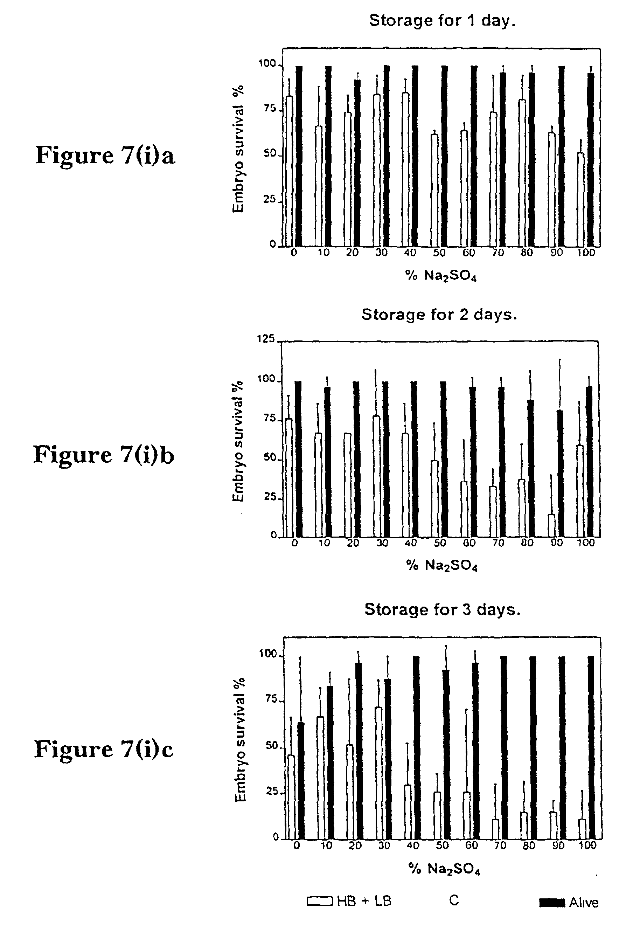

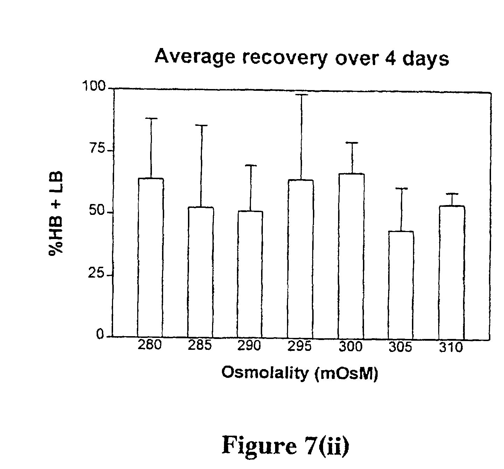

A raffinose/TMAO 1.6:1 solution with 1.75 mM CaSO₄ was then mixed in different proportions with a solution of 0.30 OsM Na₂SO₄ containing 1.75 mM CaSO₄. The percentage of mouse embryos hatching in culture following storage in these solutions for 1, 2 and 3 days are shown in Figs. 7(i)A, B and C, respectively. The highest percentage of hatched embryos was obtained with 70% raffinose/TMAO (1.6:1), 30% Na₂SO₄ and 1.75 mM CaSO₄ (hereinafter referred to as Solution 70/30). Fig. 7(ii) shows the survival of embryos following storage in Solution 70/30 of various osmolalities The optimal osmolality appears to be close to 300 mOsM but not to be of critical importance. Solution 70/30 was subsequently used for many applications and proved to be the most effective storage solution for bone marrow stem cells, hearts, red blood cells and osteoblasts. Solution 70/30 without Ca²⁺ was found to be the most effective solution for the preservation of platelets.

### Example 2

As described below, survival of mouse embryos in storage was found to be greatly enhanced by pretreatment with either a Class II solute or sodium butyrate.

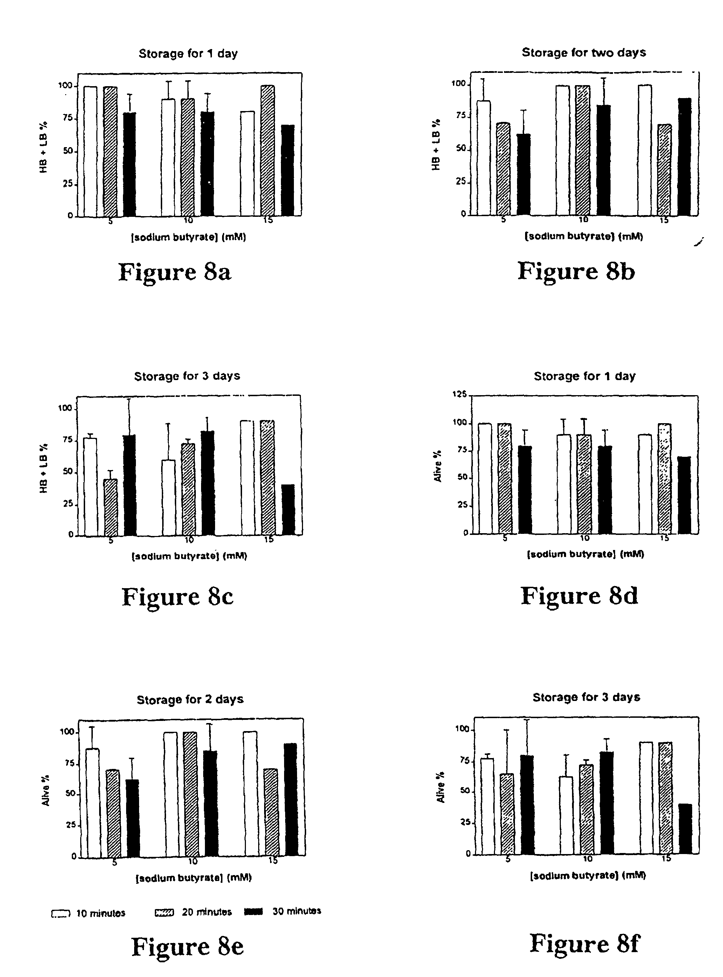

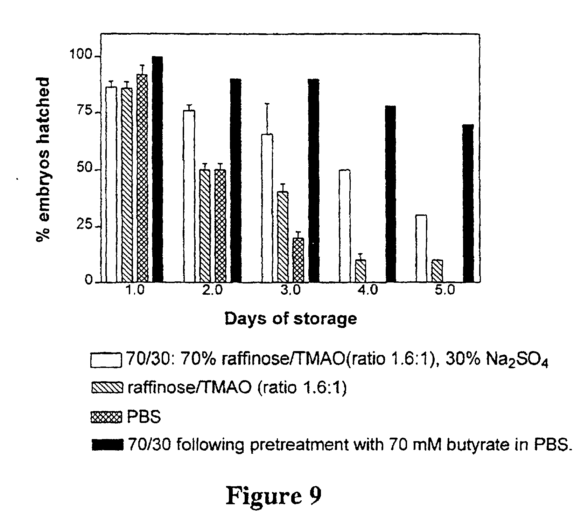

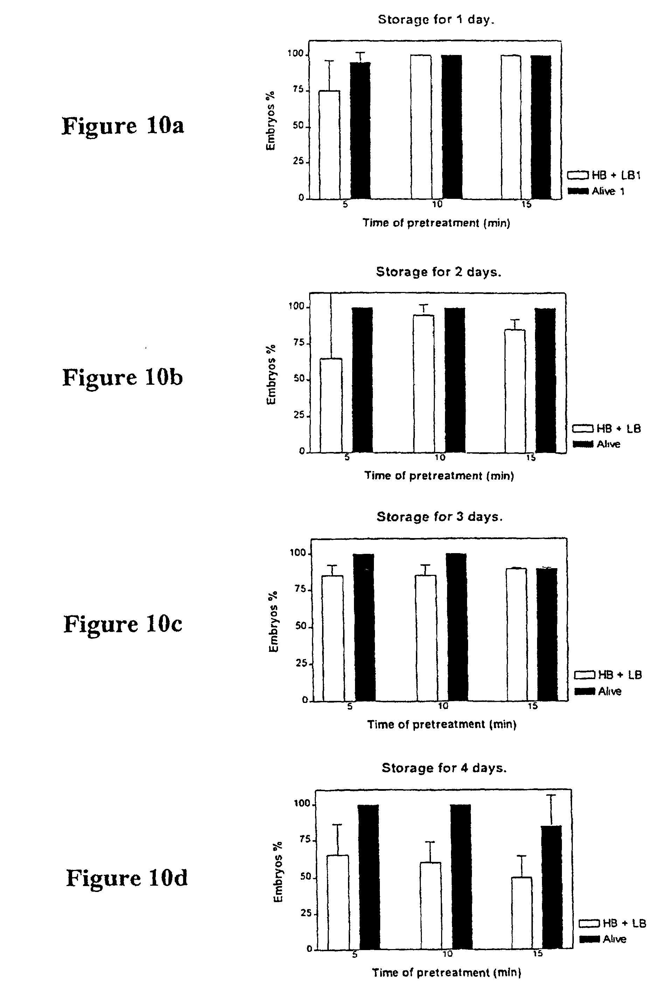

Mouse embryos were incubated with either sodium butyrate or a Class II solute at either room temperature, 30 °C or 4 °C prior to storage in Solution 70/30 for up to five days at 4 °C. Many different combinations of concentrations of sodium butyrate (5- 70 mM) and times of pretreatment (5 - 30 minutes) at room temperature gave significantly improved storage times. Sodium butyrate replaced sodium chloride at the same concentration in PBS. Figs. 8A, B and C show the percentage of mouse embryos hatching after 1, 2 and 3 days, respectively, in storage following pretreatment with sodium butyrate at concentrations of 5, 10 or 15 mM for either 10, 20 or 30 minutes. Figs. 8D, E and F show the percentage of mouse embryos alive after 1, 2 and 3 days, respectively, in storage following pretreatment with sodium butyrate at concentrations of 5, 10 or 15 mM for either 10, 20 or 30 minutes. Pretreatment with sodium butyrate allowed up to 80% of embryos to hatch following three days of storage in Solution 70/30. After 5 days of storage in Solution 70/30 following pretreatment with higher concentrations of sodium butyrate, up to 70% of embryos hatched compared to 2% with no pretreatment (see Fig. 9). Embryos stored in PBS without pretreatment lasted no longer than 3 days. Pretreatment of embryos with PBS without butyrate resulted in significant loss of embryos. Figs. 10A, B, C and D show the survival of mouse embryos after up to four days of storage in Solution 70/30 following pretreatment with 25 mM sodium butyrate for 5, 10 or 15 minutes at room temperature.

### Example 3

The efficacy of Solution 70/30 in the storage of whole blood was investigated as detailed below.

Whole blood was diluted 1:1 by volume with either plasma, Ca²⁺-containing Solution 70/30 or Ca²⁺-free Solution 70/30, and stored at 4 °C for periods of up to 28 days. In the presence of citrate-based anticoagulant solutions, platelets decreased to about 30% of their initial numbers in 18 days. When EDTA was used as the anticoagulant, platelet numbers stayed in the normal range, i.e. close to about 60% survival, in Ca²⁺-free Solution 70/30 but not in Ca²⁺-containing Solution 70/30 or plasma.

In the same tests, white cells survived little better than platelets in a citrate-based anticoagulant. Highest survival rates after 18 days were obtained when blood was collected into an EDTA containing bag and diluted 1.1 by volume with Ca²⁺-containing Solution 70/30, compared to storage in either Ca²⁺-free Solution 70/30 of plasma. This replaced the Ca²⁺ necessary for white cell storage and avoided the harmful effects of citrate.

### Example 4

This example illustrates the efficacy of the preservation solutions of the present invention in storage of isolated platelets.

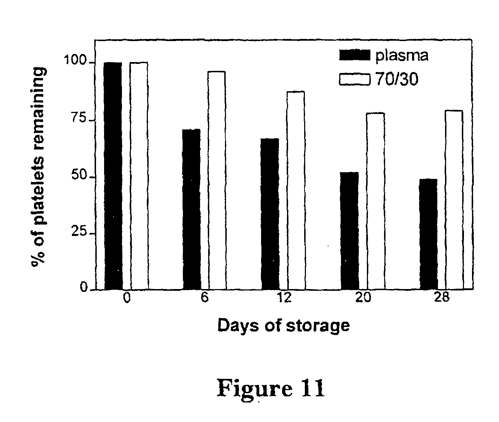

Blood was collected in EDTA and platelets isolated using standard centrifugation techniques. The final platelet-rich pellet was diluted into 50 ml of either plasma or Ca²⁺-free Solution 70/30. Fig. 11 shows that 80% of platelets survived after 28 days of storage at 4 °C. This survival rate after storage was significantly better than that in plasma and considerably better than the five days for which platelets are typically held at 21 °C. The advantages of collection of blood in EDTA and avoidance of citrate, together with storage in Ca²⁺-free Solution 70/30 at 4 °C are very clear.

### Example 5

This example illustrates the efficacy of solutions of the present invention for preservation of bone marrow.

Bone marrow was collected in heparin from two different patients and diluted 1:1 by volume with solutions of the present invention or with a standard saline solution (Hanks buffered saline solution (HBSS), or saline-based murine culture medium (M-2)). The bone marrow was stored at 4 °C for periods ranging up to 28 days, at which time the white cell count and viability, number of colony forming units, and populations of CD34 and CD45 cells were determined.

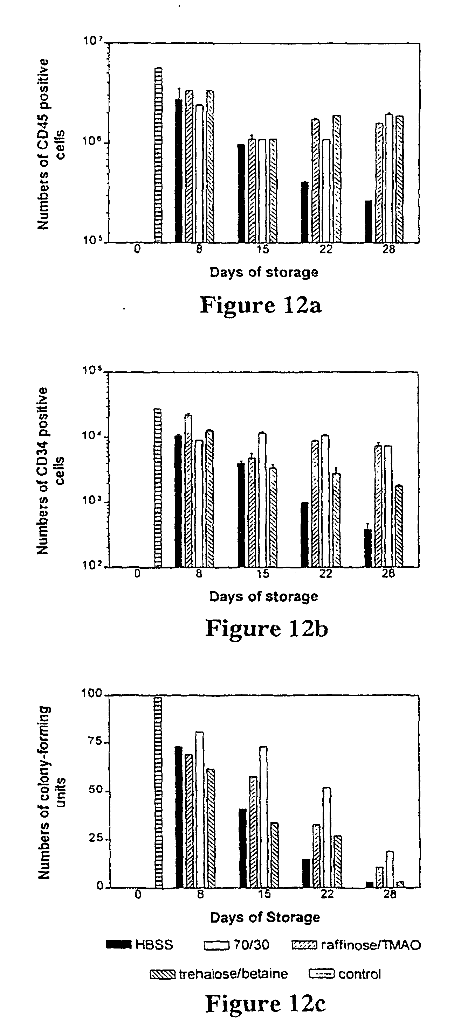

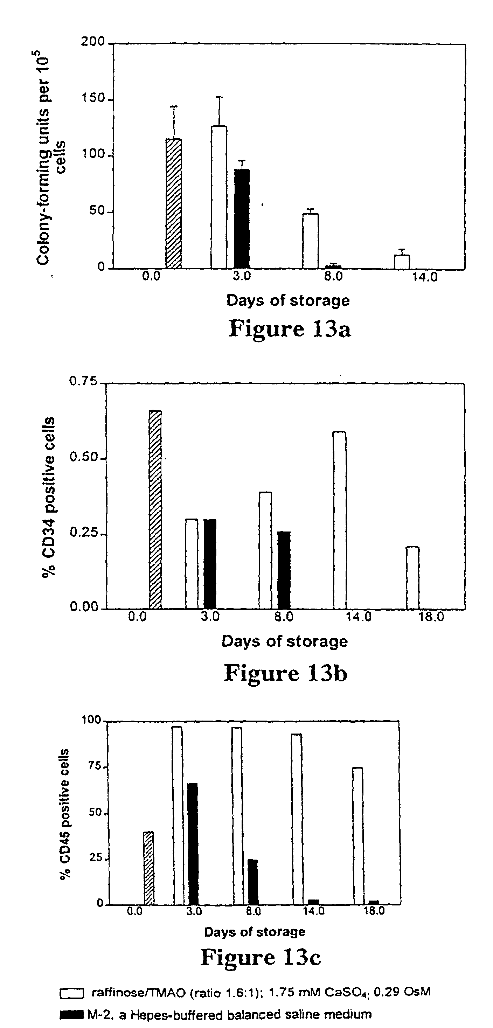

Figs. 12A, B and C show the number of CD-45 positive and CD-34 positive cells and colony-forming units, respectively, from bone marrow harvested from patient 1 and stored in the inventive solutions for up to 28 days. Figs. 13A, B and C show the number of colony-forming units, CD45-positive and CD34-positive cells, respectively, from bone marrow harvested from patient 2 and stored at 4°C in preservative and control solutions. Raffinose/TMAO had a molar ratio of 1.6:1 and the trehalose/betaine solution had a molar ratio of 1.4:1. Solution 70/30 was particularly effective in preserving bone marrow stem cells, the numbers of colony-forming units, CD45 and CD34-positive cells being much higher than they were in any of the control solutions, with the relative improvement increasing with time. Figs.13A, B and C demonstrate that the number of colony forming units, CD34-positive cells and CD-45 positive cells was significantly higher following storage in Solution 70/30 compared to storage in the saline medium M-2. The ability to store bone marrow for periods of 2-3 weeks without freezing is particularly advantageous in bone marrow transplants, since it avoids the toxicity associated with the use of DMSO in cryopreservation and allows time for a therapeutic regime, such as whole-body radiation, before re-infusion.

### Example 6

The efficacy of the inventive solutions for preservation of murine bone marrow cells was determined as follows.

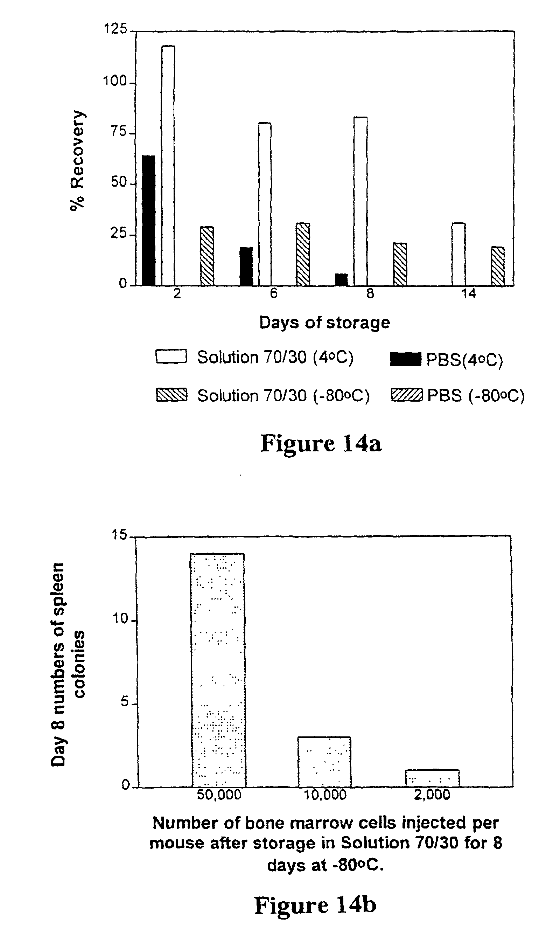

Murine bone marrow was harvested directly into Solution 70/30 or into PBS. The resulting solutions were stored either at 4°C or -80 °C. Fig. 14A shows that murine bone marrow stored in Solution 70/30 at 4°C showed 28% recovery after 14 days, with no bone marrow cells stored at 4°C in PBS for 14 days surviving. Bone marrow frozen in Solution 70/30 at -80°C showed 20% recovery after 8 and 14 days, whereas no bone marrow cells frozen in PBS at -80°C for 2, 6, 8 and 14 days survived. Fig. 14B shows that murine bone marrow frozen in Solution 70/30 at -80°C for 8 days, thawed and then injected into lethally irradiated (1000R) syngeneic mice, developed spleen colonies when analyzed eight days after injection. Mice injected with 50,000 bone marrow cells developed sixteen colonies, mice injected with 10,000 cells developed four colonies, and one mouse injected with 2000 bone marrow cells developed two colonies. These data demonstrate that murine haematopoietic stem cells survive freezing in Solution 70/30 at -80 °C for 8 days and retain *in vivo* spleen colony forming properties.

### Example 7

The efficacy of the inventive solutions for preservation of hearts was determined as follows.

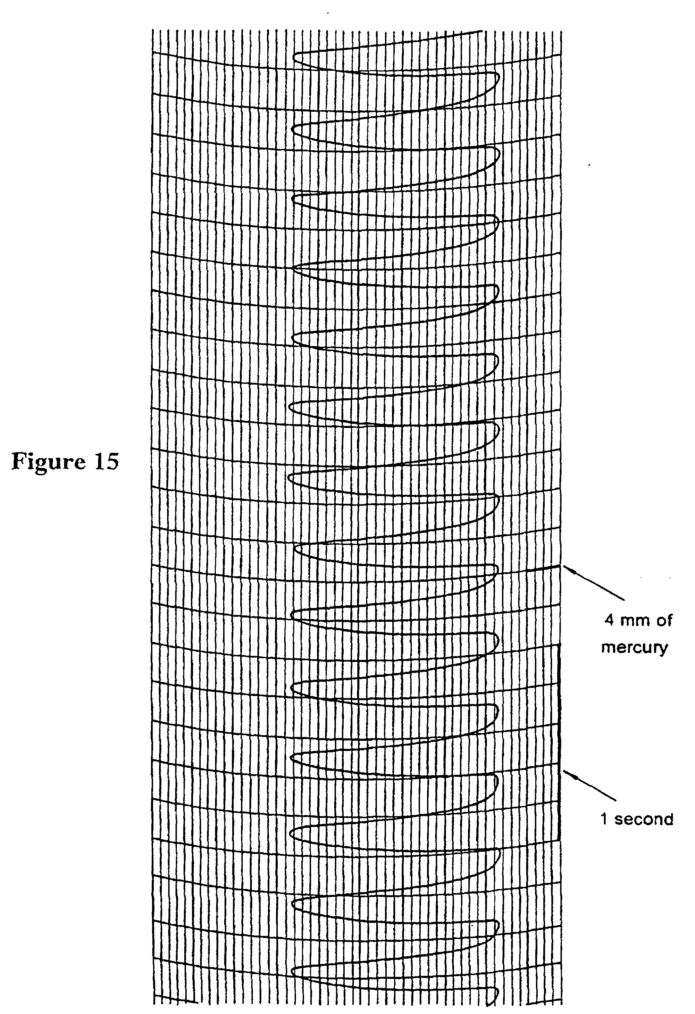

Rat hearts were surgically removed and perfused through the aorta with either Solution 70/30 or raffinose/TMAO (molar ratio 1.6:1) at 4 °C, during which time the heart rate fell from about 300 beats per minute to about 180 beats per minute. The hearts were then stored in the same solution for between 4 to 24 hours, during which time the hearts stopped beating. The hearts were subsequently remounted on a cannula and reperfused with Krebs solution initially at room temperature rising to 37 °C over 20 minutes. Using only gravity feed of the perfusing solutions, recovery of hearts after 4 hours of storage was excellent, with both heart rate and developed pressure in the normal range (heart rate 170 beats/minute, pressure 98 mm mercury; Fig. 15). When pumps were used in perfusion, variable results were obtained. In general, the pressure exerted by the pump on the heart was found to be damaging, with the damage often being irreversible.

Storage for periods longer than 4 hours was achieved by pretreating the heart with 25 mM taurine in Krebs solution for 10 minutes at 38 °C before perfusion with cold Solution 70/30 or raffinose/TMAO and storage at 4 °C. With only gravity feed for the initial perfusion and the reperfusion, hearts stored for 24 hours recovered heart rate in the normal range and pressure approximately half the normal level. Subsequent experiments showed that pretreatment with taurine could be avoided by adding approximately 0.2 mM taurine to the storage solution to prevent efflux of endogenous taurine. The results obtained using the storage solutions of the present invention compare favorably with the prior art technique of preserving hearts in cold saline-based media, wherein the heart can only be stored for 5 hours or less due to unacceptable deterioration of biological function. Storage of a heart for 24 hours without deterioration would allow time for its transport for transplantation worldwide.

### Example 8

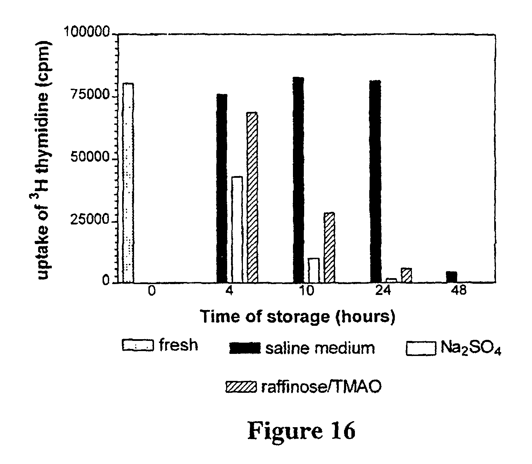

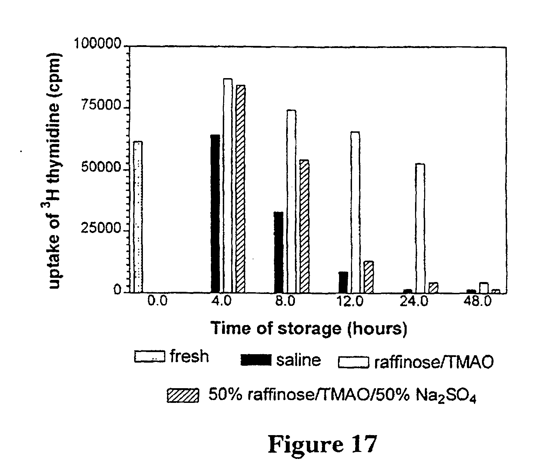

The efficacy of the inventive solutions for the preservation of various tumor cell lines, including the human lymphocytic leukemia Jurkart and K562 chronic myelogenous leukemia cell lines was tested using the solutions tested for preservation of human bone marrow described above in Examples 5 and 6. In contrast to the bone marrow progenitor cells, the tumor cell lines survived only two days in the inventive solutions before complete cell death (see Figs. 16 and 17). Thus, storage of bone marrow in the preservative solutions of the present invention for periods of greater than three days would purge the bone marrow of leukemic cells while maintaining the viability of the bone marrow.

### Example 9

The efficacy of the inventive solutions in the preservation of osteoblasts was demonstrated as follows.

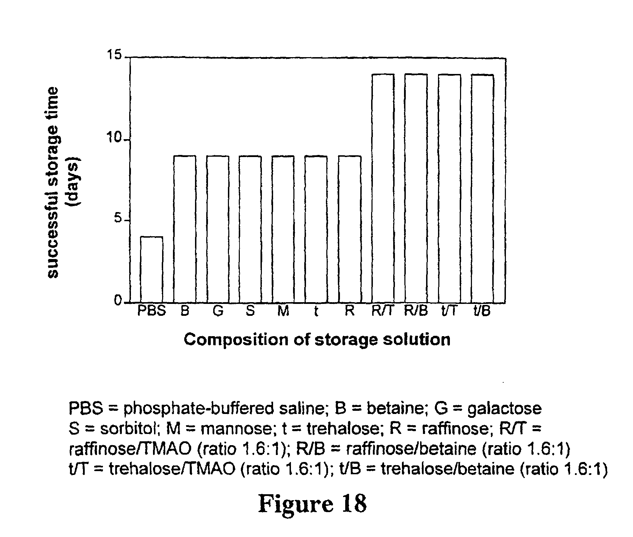

Mouse osteoblasts were dissected out and grown to near confluence in D-MEM culture medium at 38 °C. They were then dispersed with trypsin in a Ca²⁺- and Mg²⁺-free phosphate buffered saline and re-seeded into D-MEM. After further culture, the medium was removed by aspiration and replaced with one of the following solutions: PBS, betaine, galactose, sorbitol, mannose, trehalose, raffinose, raffinose/TMAO (ratio 1.6:1), raffinose/betaine (ratio 1.6:1), trehalose/TMAO (ratio 1.6:1) and trehalose/betaine (ratio 1.6:1). After storage at 4 °C for varying time intervals, the storage solution was aspirated off and replaced with D-MEM. A successful storage was one in which osteoblasts subsequently grew to confluence. As shown in Fig. 18, osteoblasts survived storage in the inventive solutions for much longer periods than in PBS. Osteoblasts were found to be more tolerant of fluctuations in osmolality than were embryos.

### Example 10

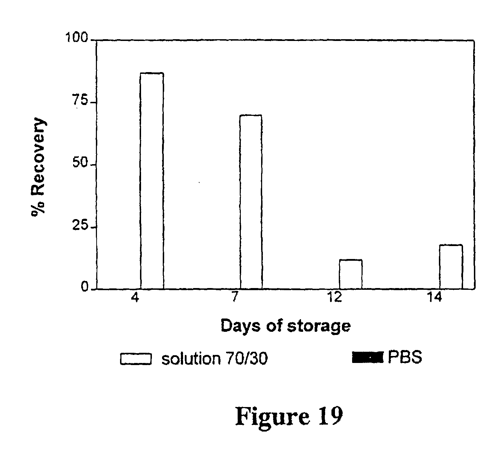

The efficacy of the inventive solutions in the preservation of murine T7T keratinocyte tumor cell line was investigated as follows. The culture medium was removed from adherent cultures of T7T growing in D-MEM supplemented with 5% serum by aspiration and replaced with PBS or Solution 70/30 prior to storage at 4 °C. After 4, 7, 12 and 14 days these solutions were removed, the adherent cells removed by trypsinization and recovery determined. Fig. 19 shows that no viable T7T cells survived in PBS but viable T7T cells were recovered following up to 14 days of storage in Solution 70/30.

### Example 11

The efficacy of the inventive solutions in the preservation of murine 3T3 fibroblast cells was demonstrated as follows.

Adherent cultures of 3T3 cells growing in D-MEM supplemented with 5% serum had the medium removed by aspiration and replaced with PBS or Solution 70/30 prior to storage at 4 °C. After 1, 7 and 14 days, these solutions were removed, the adherent cells were removed by trypsinization and recovery was determined. As shown in Fig. 19, no viable 3T3 cells survived in PBS but viable 3T3 cells were recovered after up to 14 days of storage in Solution 70/30.

Although the present invention has been described in terms of specific embodiments, changes and modifications can be carried out without departing from the scope of the invention which is intended to be limited only by the scope of the appended claims.

## Claims

1. A solution for the preservation of living biological materials, comprising:
(a) a first solute selected from the group consisting of raffinose and trehalose, and
(b) a second solute selected from the group consisting, of trimethyl amine oxide and betaine,
wherein the solution is isotonic with the material to be preserved and the solution has a concentration of iodide, dihydrogen phosphate, bicarbonate, nitrate and bisulfate that is less than a concentration sufficient to increase the metabolic activity of the biological material during preservation.

2. The solution of claim 1 for the preservation of living biological materials comprising raffinose as a first solute and trimethyl amine oxide as a second solute.

3. The solution of either of claims 1 and 2, wherein the living biological material to be preserved is a mamalian material and the solution has an osmolality of about 280 mOsM to about 320 mOsM.

4. The solution of either of claims 1 and 2, wherein the living biological material to be preserved is a plant material and the solution has an osmolality of about 70 mOsM to about 80 mOsM.

5. The solution of either of claims 1 and 2, wherein the living biological material to be preserved is a marine material and the solution has an osmolality of about 900 mOsM to about 1000 mOsM.

6. The solution of claim 2, wherein the raffinose and trimethyl amine oxide are present in a molar ratio of about 1.1 to 1 to about 2.0 to 1.

7. The solution of claim 2, wherein the raffinose and trimethyl amine oxide are present in a molar ratio of about 1.4 to 1 to about 1.8 to 1.

8. The solution of claim 2 further comprising calcium ions.

9. The solution of claim 8 wherein the calcium ions are from a compound selected from the group consisting of calcium sulfate and calcium chloride.

10. The solution of either of claims 2 and 8 further comprising sodium sulfate.

11. A solution for the preservation of living mammalian biological materials comprising
(a) raffinose and trimethyl amine oxide in a molar ratio of about 1.1 to 1 to about 2.0 to 1; and
(b) sodium sulfate,
wherein the solution has an osmolality of about 280 mOsM to about 320 mOsM and the solution has a concentration of iodide, dihydrogen phosphate, bicarbonate, nitrate and bisulfate that is less than a concentration sufficient to increase metabolic activity of the biological material during preservation.

12. The solution of claim 1 for the preservation of living biological materials either comprising:
(a) trehalose and trimethyl amine oxide in a molar ratio of about 1.1 to 1 to about 1.4 to 1;
(b) raffinose and betaine in a molar ratio of about 1.4 to 1 to about 1.6 to 1; or
(c) trehalose and betaine in a molar ratio of about 1.4 to 1 to about 1.6 to 1;
wherein the solution is isotonic with the material to be preserved and the solution has a concentration of iodide, dihydrogen phosphate, bicarbonate, nitrate and bisulfate that is less than a concentration sufficient to increase the metabolic activity of the biological material during preservation.

13. A concentrated liquid solution that, upon addition of an aqueous liquid, forms a solution according to any one of claims 1, 2, 11 and 12.

14. A collection of solids that upon addition of an aqueous liquid, forms a solution according to any one of claims 1, 2, 11 and 12.

15. A method for preserving the viability of a biological material, comprising contacting the material with a solution according to any one of claims 1, 2, 11 and 12.

16. A method for preserving the viability of a living biological material, comprising:
(a) contacting, the biological material with a solution according to any one of claims 1, 2, 11 and 12; and
(b) maintaining the biological material at a temperature less than about 4 °C.

17. A method for preserving the viability of a living mammalian biological material, comprising contacting the biological material with a solution according to any one of claims 8 and 11.

18. The method of claim 17, wherein the living mammalian biological material is selected from the group consisting of organs, tissues and cells.

19. The method of claim 17 wherein the living mammalian biological material is selected from the group consisting of: heart, stem cells, bone marrow, embryos, platelets, osteoblasts and skin cells.

20. A method for preserving the viability of a living plant biological material, comprising contacting the biological material with a solution according to claim 9.

21. A method for preserving the viability of a living marine biological material, comprising contacting the biological material with a solution according to claim 10.

22. The method of claim 20, wherein the preservative solution comprises:
(a) a first solute selected frorn the group consisting of raffinose and trehalose; and
(b) a second solute selected from the group consisting of trimethyl amine oxide and betaine,
the solution being isotonic with the material to be preserved and the solution having a concentration of iodide, dihydrogen phosphate, bicarbonate, nitrate and bisulfate that is less than a concentration sufficient to increase the metabolic activity of the biological material during preservation.

## Patentansprüche

1. Lösung für die Konservierung von lebenden biologischen Materialien, welche umfasst:
(a) einen ersten gelösten Stoff ausgewählt aus der Gruppe bestehend aus Raffinose und Trehalose, und
(b) einen zweiten gelösten Stoff ausgewählt aus der Gruppe bestehend aus Trimethylaminoxid und Betain,
worin die Lösung und das zu konservierenden Material isoton sind und die Lösung eine Konzentration an Iod, Dihydrogenmonophosphat, Hydrogencarbonat, Nitrat und Hydrogensulfat aufweist, die niedriger als diejenige Konzentration ist, die ausreicht, um die metabolische Aktivität des biologischen Materials während der Konservierung zu erhöhen.

2. Lösung nach Anspruch 1 zur Konservierung von lebenden biologischen Materialien, welche Raffinose als ersten gelösten Stoff und Trimethylaminoxid als zweiten gelösten Stoff umfasst.

3. Lösung nach einem der Ansprüche 1 und 2, worin das zu konservierende lebende biologische Material Säugetiermaterial ist und die Lösung eine Osmolalität von ungefähr 280 mOsM bis ungefähr 320 mOsM besitzt.

4. Lösung nach einem der Ansprüche 1 und 2, worin das zu konservierende lebende biologische Material Pflanzenmaterial ist und die Lösung eine Osmolalität von ungefähr 70 mOsM bis ungefähr 80 mOsM besitzt.

5. Lösung nach einem der Ansprüche 1 und 2, worin das zu konservierende lebende biologische Material marines Material ist und die Lösung eine Osmolalität von ungefähr 900 mOsM bis ungefähr 1000 mOsM besitzt.

6. Lösung nach Anspruch 2, worin die Raffinose und das Trimethylaminoxid in einem molaren Verhältnis von ungefähr 1,1 zu 1 bis ungefähr 2,0 zu 1 vorhanden sind.

7. Lösung nach Anspruch 2, worin die Raffinose und das Trimethylaminoxid in einem molaren Verhältnis von ungefähr 1,4 zu 1 bis ungefähr 1,8 zu 1 vorhanden sind.

8. Lösung nach Anspruch 2, welche weiterhin Kalziumionen umfasst.

9. Lösung nach Anspruch 8, worin die Kalziumionen von einer Verbindung ausgewählt aus der Gruppe bestehend aus Kalziumsulfat und Kalziumchlorid stammen.

10. Lösung nach einem der Ansprüche 2 und 8, welche weiterhin Natriumsulfat umfasst.

11. Lösung für die Konservierung von lebenden biologischen Säugetiermaterialien, welche umfasst:
(a) Raffinose und Trimethylaminoxid in einem molaren Verhältnis von ungefähr 1,1 zu 1 bis ungefähr 2,0 zu 1; und
(b) Natriumsulfat,
worin die Lösung eine Osmolalität von ungefähr 280 mOsM bis ungefähr 320 mOsM besitzt und die Lösung eine Konzentration von Iod, Dihydrogenmonophosphat, Hydrogencarbonat, Nitrat und Hydrogensulfat aufweist, die niedriger als diejenige Konzentration ist, die ausreicht, um die metabolische Aktivität des biologischen Materials während der Konservierung zu erhöhen.

12. Lösung nach Anspruch 1 für die Konservierung von lebenden biologischen Materialien, die entweder
(a) Trehalose und Trimethylaminoxid in einem molaren Verhältnis von ungefähr 1,1 zu 1 bis ungefähr 1,4 zu 1;
(b) Raffinose und Betain in einem molaren Verhältnis von ungefähr 1,6 zu 1; oder
(c) Trehalose und Betain in einem molaren Verhältnis von ungefähr 1,4 zu 1 bis ungefähr 1,6 zu 1 umfasst;
worin die Lösung mit dem zu konservierenden Material isoton ist und die Lösung eine Konzentration von Iod, Dihydrogenmonophosphat, Hydrogencarbonat, Nitrat und Hydrogensulfat aufweist, die niedriger als diejenige Konzentration ist, die ausreicht, um die metabolische Aktivität des biologischen Materials während der Konservierung zu erhöhen.

13. Konzentrierte flüssige Lösung, die nach Zugabe einer wässrigen Flüssigkeit eine Lösung nach einem der Ansprüche 1, 2, 11 und 12 ergibt.

14. Sammlung von Feststoffen, die nach Zugabe einer wässrigen Flüssigkeit eine Lösung nach einem der Ansprüche 1, 2, 11 und 12 ergibt.

15. Verfahren für die Konservierung der Lebensfähigkeit eines biologischen Materials, welches ein in-Kontakt-bringen des Materials mit einer Lösung nach einem der Ansprüche 1, 2, 11 und 12 umfasst.

16. Verfahren für die Konservierung der Lebensfähigkeit eines lebenden biologischen Materials, welches umfasst:
(a) in-Kontakt-bringen des Materials mit einer Lösung nach einem der Ansprüche 1, 2, 11 und 12; und
(b) Aufbewahren des biologischen Materials bei einer Temperatur niedriger als ungefähr 4°C.

17. Verfahren zur Konservierung der Lebensfähigkeit eines lebenden biologischen Säugetiermaterials, welches das in-Kontakt-bringen des biologischen Materials mit einer Lösung nach einem der Ansprüche 8 und 11 umfasst.

18. Verfahren nach Anspruch 17, worin das lebende biologische Säugetiermaterial ausgewählt wird aus der Gruppe bestehend aus Organen, Geweben und Zellen.

19. Verfahren nach Anspruch 17, worin das lebende biologische Säugetiermaterial ausgewählt wird aus der Gruppe bestehend aus: Herz, Stammzellen, Knochenmark, Embryonen, Blutplättchen, Osteoblasten und Hautzellen.

20. Verfahren für die Konservierung der Lebensfähigkeit von lebendem biologischen Pflanzenmaterial, welches das in-Kontakt-bringen des biologischen Materials mit einer Lösung nach Anspruch 9 umfasst.

21. Verfahren für die Konservierung der Lebensfähigkeit von lebendem biologischen marinem Material, welches das in-Kontakt-bringen des biologischen Materials mit einer Lösung nach Anspruch 10 umfasst.

22. Verfahren nach Anspruch 20, worin die Konservierungslösung
(a) einen ersten gelösten Stoff ausgewählt aus der Gruppe bestehend aus Raffinose und Trehalose; und
(b) einen zweiten gelösten Stoff ausgewählt aus der Gruppe bestehend aus Trimethylaminoxid und Betain umfasst,
wobei die Lösung mit dem zu konservierenden Material isoton ist, und die Lösung eine Konzentration von Iod, Dihydrogenmonophosphat, Hydrogencarbonat, Nitrat und Hydrogensulfat aufweist, die niedriger als diejenige Konzentration ist, die ausreicht, um die metabolische Aktivität des biologischen Materials während der Konservierung zu erhöhen.

## Revendications

1. Solution destinée à la conservation de matériaux biologiques vivants, comprenant :
(a) un premier soluté choisi parmi le groupe constitué du raffinose et du tréhalose, et
(b) un second soluté choisi parmi le groupe constitué de l'oxyde de triméthylamine et de la bétaïne,
dans laquelle la solution est isotonique avec le matériau devant être conservé et la solution présente une concentration en iodure, en dihydrogénophosphate, en bicarbonate, en nitrate et en bisulfate qui est inférieure à une concentration suffisante pour augmenter l'activité métabolique du matériau biologique durant la conservation.

2. Solution selon la revendication 1 destinée à la conservation de matériaux biologiques vivants, comprenant du raffinose en tant que premier soluté et de l'oxyde de triméthylamine en tant que second soluté.

3. Solution selon l'une ou l'autre des revendications 1 et 2, dans laquelle le matériau biologique vivant devant être conservé est un matériau de mammifère et la solution présente une osmolalité d'environ 280 mOsM à environ 320 mOsM.

4. Solution selon l'une ou l'autre des revendications 1 et 2, dans laquelle le matériau biologique vivant devant être conservé est un matériau végétal et la solution présente une osmolalité d'environ 70 mOsM à environ 80 mOsM.

5. Solution selon l'une ou l'autre des revendications 1 et 2, dans laquelle le matériau biologique vivant devant être conservé est un matériau marin et la solution présente une osmolalité d'environ 900 mOsM à environ 1 000 mOsM.

6. Solution selon la revendication 2, dans laquelle le raffinose et l'oxyde de triméthylamine sont présents suivant un rapport molaire d'environ 1,1 sur 1 à environ 2,0 sur 1.

7. Solution selon la revendication 2, dans laquelle le raffinose et l'oxyde de triméthylamine sont présents suivant un rapport molaire d'environ 1,4 sur 1 à environ 1,8 sur 1.

8. Solution selon la revendication 2, comprenant en outre des ions calcium.

9. Solution selon la revendication 8, dans laquelle les ions calcium proviennent d'un composé choisi parmi le groupe constitué du sulfate de calcium et du chlorure de calcium.

10. Solution selon l'une ou l'autre des revendications 2 et 8, comprenant en outre du sulfate de sodium.

11. Solution destinée à la conservation de matériaux biologiques de mammifères vivants comprenant
(a) du raffinose et de l'oxyde de triméthylamine suivant un rapport molaire d'environ 1,1 sur 1 à environ 2,0 sur 1, et
(b) du sulfate de sodium,
dans laquelle la solution présente une osmolalité d'environ 280 mOsM à environ 320 mOsM et la solution présente une concentration en iodure, en dihydrogénophosphate, en bicarbonate, en nitrate et en bisulfate qui est inférieure à une concentration suffisante pour augmenter l'activité métabolique du matériau biologique durant la conservation.

12. Solution selon la revendication 1, destinée à la conservation de matériaux biologiques vivants comprenant soit :
(a) du tréhalose et de l'oxyde de triméthylamine suivant un rapport molaire d'environ 1,1 sur 1 à environ 1,4 sur 1,
(b) du raffinose et de la bétaïne suivant un rapport molaire d'environ 1,4 sur 1 à environ 1,6 sur 1, soit
(c) du tréhalose et de la bétaïne suivant un rapport molaire d'environ 1,4 sur 1 à environ 1,6 sur 1,
dans laquelle la solution est isotonique avec le matériau devant être conservé et la solution présente une concentration en iodure, en dihydrogénophosphate, en bicarbonate, en nitrate et en bisulfate qui est inférieure à une concentration suffisante pour augmenter l'activité métabolique du matériau biologique durant la conservation.

13. Solution liquide concentrée qui, lors de l'addition d'un liquide aqueux, forme une solution selon l'une quelconque des revendications 1, 2, 11 et 12.

14. Ensemble de solides qui, lors de l'addition d'un liquide aqueux, forme une solution selon l'une quelconque des revendications 1, 2, 11 et 12.

15. Procédé de conservation de la viabilité d'un matériau biologique, comprenant la mise en contact du matériau avec une solution selon l'une quelconque des revendications 1, 2, 11 et 12.

16. Procédé de conservation de la viabilité d'un matériau biologique vivant, comprenant :
(a) la mise en contact du matériau biologique avec une solution selon l'une quelconque des revendications 1, 2, 11 et 12, et
(b) le maintien du matériau biologique à une température inférieure à environ 4 °C.

17. Procédé de conservation de la viabilité d'un matériau biologique de mammifère vivant, comprenant la mise en contact du matériau biologique avec une solution selon l'une quelconque des revendications 8 et 11.

18. Procédé selon la revendication 17, dans lequel le matériau biologique de mammifère vivant est choisi parmi le groupe constitué d'organes, de tissus et de cellules.

19. Procédé selon la revendication 17, dans lequel le matériau biologique de mammifère vivant est choisi parmi le groupe constitué: du coeur, des cellules souches, de la moelle osseuse, des embryons, des plaquettes, des ostéoblastes et des cellules cutanées.

20. Procédé de conservation de la viabilité d'un matériau biologique végétal vivant, comprenant la mise en contact du matériau biologique avec une solution selon la revendication 9.

21. Procédé de conversation de la viabilité d'un matériau biologique marin vivant, comprenant la mise en contact du matériau biologique avec une solution selon la revendication 10.

22. Procédé selon la revendication 20, dans lequel la solution de conservation comprend :
(a) un premier soluté choisi parmi le groupe constitué du raffinose et du tréhalose, et
(b) un second soluté choisi parmi le groupe constitué de l'oxyde de triméthylamine et de la bétaïne,
la solution étant isotonique avec le matériau devant être conservé et la solution présentant une concentration en iodure, en dihydrogénophosphate, en bicarbonate, en nitrate et en bisulfate qui est inférieure à une concentration suffisante pour augmenter l'activité métabolique du matériau biologique durant la conservation.
